(19) Europäisches Patentamt / European Patent Office / Office européen des brevets

(11) **EP 3 300 718 B1**

(12) **EUROPEAN PATENT SPECIFICATION**

(45) Date of publication and mention
of the grant of the patent:
**01.05.2019 Bulletin 2019/18**

(21) Application number: **16191263.9**

(22) Date of filing: **29.09.2016**

(51) Int Cl.:
*A61K 8/55* *(2006.01)*        *A61K 8/73* *(2006.01)*
*A61Q 19/00* *(2006.01)*      *A61K 8/04* *(2006.01)*
*A61K 8/06* *(2006.01)*        *A61K 8/44* *(2006.01)*
*A61K 8/60* *(2006.01)*        *A61K 8/37* *(2006.01)*

(54) **LOW DENSITY, STABLE FOAM FOR TOPICAL APPLICATION**

STABILER SCHAUM MIT GERINGER DICHTE ZUR TOPISCHEN ANWENDUNG

MOUSSE STABLE DE FAIBLE DENSITE POUR APPLICATION TOPIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**04.04.2018 Bulletin 2018/14**

(73) Proprietor: **Cargill, Incorporated
Wayzata, MN 55391 (US)**

(72) Inventor: **TRESSON, Barbara Karin Marie
1800 Vilvoorde (BE)**

(74) Representative: **Morariu Radu, Mihai Dorin
Cargill R&D Centre Europe BVBA
Bedrijvenlaan 9
2800 Mechelen (BE)**

(56) References cited:
**WO-A1-2015/096115     KR-A- 20110 056 877**

**Description**

FIELD OF THE INVENTION

The present invention relates to a foamed composition suitable for topical application, said composition comprising a fatty phase dispersed in an aqueous phase, at least one starch phosphate and a surfactant. The invention also relates to the use of said composition in the cosmetic and dermatological fields, in particular for caring for or treating keratin materials.

BACKGROUND OF THE INVENTION

In the cosmetic field, and more particularly in the field of skincare, photoprotection or makeup, it is known practice to use various ingredients such as starch phosphates in the preparation of oil-in-water or water-in-oil emulsions to give them suitable textures and advantageous sensory effects such as creaminess and cocooning.

In the latest years however, creativity gained a predominant role with texture being one of the factors of choice for seducing the consumer. Softness, lightness, surprising sensory effects and original feels are continuously evolving. Nowadays, beyond the performance of cosmetic care products, cosmetic product consumers are continually searching for and attempting to develop new sensations on the skin.

Amongst the newly developed cosmetic products, those in the form of a foam seemed very promising. Although foamed products such as "shaving foams" and "hair styling foams" already existed for a long time, these technologies never concretize themselves into a galenic form for the cosmetics market. Their main drawback was their reduced stability and the various packaging challenges since such "shaving of hair-styling foams" could not be packaged in a jar.

The main drawback of foamed cosmetics products can be defined as the technical difficulty in obtaining a product in a jar, which is both stable over time and provides optimum sensoriality, e.g. lightness, spreading, softness, comfort and pleasure. Rather recently, foams packaged in a jar, such as "Calendula Cream" from Laboratoires Boiron ®, appeared on the market, however, they are intended for treating erythema, irritated skin, dry patches, cracks and chapping, and were not designed to have satisfactory cosmetic properties e.g. in terms of sensoriality. They also seem to have a reduced stability since their reported shelve life is shorter than six months.

Other foams intended for the cosmetic market which seemingly have reasonable stability are known from WO 2013/087926 and WO 2015/096115. However, their properties in terms of sensoriality and stability can be further improved.

There remains thus a need for a cosmetic compositions in the form of a foam which can be easily packaged in a jar and be stable over time and to temperature variations. In particular there is a need for cosmetic foams with a reduced density, e.g. a density at 20°C ranging from 0.4 to 0.9, which can be spread easily on a keratin material such as the skin and produce, on application and even after being applied, a feeling of softness and creaminess, showing non-tackiness and which can be suitable for a wide spectrum of cosmetic applications.

SUMMARY OF THE INVENTION

In an attempt to respond to the above mentioned needs, the present inventors propose a composition comprising a fatty phase dispersed in an aqueous phase, said composition being in the form of a foam and having a density of between 0.4 to 0.9, the composition comprising: (a) at least one starch phosphate; (b) Xanthan Gum (XG); (c) Locust Bean Gum (LBG); and (d) at least one surfactant, wherein the XG and the LBG are in a ratio XG:LBG from 20:80 to 80:20;

The present inventors observed that the composition of the invention, hereinafter *"the inventive composition"*, shows an excellent sensory texture and provides a novel sensory feeling. In particular they observed that when applied on e.g. the skin, the application is accompanied by a "cracking" sound coming from the bursting of air pockets contained by the foam. Due to an optimization of the size and the density of the air pockets, the "cracking" sound is profound and subtle while the bursting of the air pockets gives a delicate, gentle and soft sensation on the skin. Such sensory effects are unique not being provided hitherto to inventors' knowledge by any known cosmetic foamed or non-foamed product. In addition, the inventive composition spreads uniformly on the skin and does not pill after application.

Moreover, the inventive composition is stable at both low temperature and high temperature for prolonged period of times and can be used in a wide range of cosmetic products for caring for and/or making up keratin materials and in particular the skin. Other benefits and advantages of the present invention will become apparent from the disclosure given hereinbelow.

DETAILED DESCRIPTION OF THE INVENTION

The present invention offers a composition comprising a fatty phase dispersed in an aqueous phase, said composition being in the form of a foam and having a density of between 0.4 to 0.9, the composition comprising: (a) at least one starch phosphate; (b) Xanthan Gum (XG); (c) Locust Bean Gum (LBG); and (d) at least one surfactant, wherein the XG and the LBG are in a ratio XG:LBG from 20:80 to 80:20;

One intended use of the inventive composition is in topical application to the skin or the integuments. For such a use, said composition preferably comprises a physiologically acceptable medium, i.e. a medium that is compatible with all keratin materials, such as the skin, nails, mucous membranes and keratin fibers (such as the hair or the eyelashes). Preferably, the cosmetically acceptable medium has a pleasant color, odor and feel and does not cause any unacceptable discomfort (stinging, tautness or redness) liable to dissuade the consumer from using the composition.

The density of the inventive composition is preferably at least 0.3, more preferably at least 0.4, most preferably at least 0.5. Said density is preferably at most 0.9, more preferably at most 0.8, most preferably at most 0.7. Said density preferably ranges from 0.3 to 0.9 and preferably from 0.4 to 0.8, more preferably between 0.5 and 0.7. Unless expressly mentioned otherwise, the density is expressed in the context of the present invention in $g/cm^3$.

The inventive composition contains an aqueous phase. The aqueous phase may be water, in particular a demineralized water; a floral water such as cornflower water; a mineral water such as Vittel water, Lucas water or La Roche Posay water; and/or a spring water. Preferably, demineralized water is used as the aqueous phase utilized by the present invention.

The aqueous phase is preferably present in the inventive composition in an amount of at least 55 wt%, more preferably at least 60 wt%, most preferably at least 65 wt%, relative to the total amount of the composition. Preferably said amount is at most 95 wt%, more preferably at most 90 wt%, most preferably at most 85 wt%. Preferably, the aqueous phase is present in an amount of between 55 wt% and 95 wt%, more preferably between 60 wt% and 90 wt%, most preferably between 65 wt% and 85 wt%.

The aqueous phase may comprise at least one hydrophilic solvent, for instance substantially linear or branched lower monoalcohols having from 1 to 8 carbon atoms, such as ethanol, propanol, butanol, isopropanol or isobutanol; polyols, such as propylene glycol, isoprene glycol, butylene glycol, glycerol, sorbitol, polyethylene glycols and derivatives thereof; and mixtures thereof.

Preferably, the aqueous phase comprises a polyol which is miscible with water at ambient temperature (25°C) chosen in particular from polyols having in particular from 2 to 20 carbon atoms, preferably having from 2 to 10 carbon atoms and preferentially having from 2 to 6 carbon atoms, such as glycerol, propylene glycol, butylene glycol, pentylene glycol, hexylene glycol, dipropylene glycol or diethylene glycol; glycol ethers (having in particular from 3 to 16 carbon atoms), such as mono-, di- or tripropylene glycol $(C_1 - C_4)$alkyl ethers, or mono-, di- or triethylene glycol $(C_1 - C_4)$alkyl ethers; and mixtures thereof.

A polyol that is suitable for the invention may be a compound of linear, branched or cyclic, saturated or unsaturated alkyl type, bearing on the alkyl chain at least two -OH functions, in particular at least three -OH functions.

The polyols that are advantageously suitable for formulating a composition according to the present invention are those containing in particular from 2 to 32 carbon atoms and preferably 3 to 16 carbon atoms. Advantageously, the polyol will be chosen from glycols, such as propylene glycols, polyethylene glycols, PEG/PPG/Polybutylene Glycol-8/5/3 Glycerin, for instance the product sold under the trade name Wilbride by the company Nof Corporation, glycerol, propylene glycol, pentylene glycol, butylene glycol, propanediol, ethylhexyl glycerol, caprylyl glycol or mixtures thereof. The polyol(s) is (are) preferably present in an amount ranging from 0.1% to 30 % and better still from 1% to 20%, by weight, relative to the total weight of said composition.

The inventive composition also contains a fatty phase dispersed in the aqueous phase, by dispersion being herein meant that the fatty phase forms droplets inside the aqueous phase. The droplets may have any sizes and shapes and they are preferably homogeneously distributed throughout the aqueous phase. The nature of the fatty phase of the composition is not critical. The fatty phase may thus consist of any fatty substance conventionally used in the cosmetic or dermatological fields; it in particular comprises at least one oil, i.e. any fatty substance that is in liquid form at room temperature (20-25°C) and at atmospheric pressure (760 mmHg).

The preferred fatty phase(s) comprises at least one oil which can be a hydrocarbon-based oil, i.e. an oil mainly containing hydrogen and carbon atoms and optionally oxygen, nitrogen, sulfur and/or phosphorus atoms, for example in the form of hydroxyl or acid radicals; a silicone oil, i.e. an oil comprising at least one silicon atom and preferably at least one Si-O group; a fluoro oil, i.e. an oil comprising at least one fluorine atom; a non-fluoro oil, or a mixture thereof. Preferably, the inventive composition comprises at least one hydrocarbon-based oil as the fatty phase.

The hydrocarbon-based oils may be of animal origin or of vegetable origin, such as liquid triglycerides of fatty acids comprising from 4 to 10 carbon atoms, such as heptanoic or octanoic acid triglycerides, or alternatively, for example, sunflower oil; maize oil; soybean oil; cucumber oil; grape seed oil; sesame seed oil; hazelnut oil; apricot oil; macadamia oil; arara oil; castor oil; cocoa butter; almond oil; avocado oil; caprylic/capric acid triglycerides, such as those sold by Stearineries Dubois or those sold under the names Miglyol 810, 812 and 818 by Dynamit Nobel; Simmondsia Chinensis (Jojoba) Seed oil sold under the tradename Jojoba Oil Golden by Desert Whale; Beta-carotene sold under the tradename Betatene 30% OLV by Cognis (BASF); Rosa Canina Fruit Oil sold under the tradename Rosehip Seed Oil by Nestle World Trade Co.; shea butter oil; and mixtures thereof. Preferably, the fatty phase contains a vegetable oil and /or a vegetable fat; more preferably it contains cocoa butter and a vegetable oil, e.g. almond oil; even more preferably, the fatty phase contains caprylic/capric acid triglycerides, cocoa butter and a vegetable oil different that said triglycerides, e.g. almond oil.

The hydrocarbon-based oils may be linear or branched hydrocarbons of mineral or synthetic origin. Alternatively, the hydrocarbon-based oils may be synthetic ethers; synthetic esters; fatty alcohols that are liquid at room temperature, with a branched and /or unsaturated carbon-based chain containing from 12 to 26 carbon atoms; $C_{12}$-$C_{22}$ higher fatty acids; or mixtures thereof.

The fatty phase is preferably present in the inventive composition in an amount of at least 5 wt%, more preferably at least 10 wt%, most preferably at least 15 wt%, relative to the total amount of the composition. Preferably said amount is at most 40 wt%, more preferably at most 35 wt%, most preferably at most 30 wt%. Preferably, the fatty phase is present in an amount of between 5 wt% and 40 wt%, more preferably between 10 wt% and 35 wt%, most preferably between 15 wt% and 30 wt%.

The composition of the present invention comprises at least one starch phosphate, or a mixture thereof. The starch phosphates are macromolecules in the form of polymers formed from hydroglucose units. The number of these units and their assembly make it possible to distinguish between amylose (linear polymer) and amylopectin (branched polymer). The relative proportions of amylose and of amylopectin, and their degree of polymerization, vary as a function of the origin of the starches. The starch molecules used in the present invention may originate from a plant source such as cereals, tubers, roots, legumes and fruit; preferably, the starch(es) originate from a plant source chosen from corn, pea, potato, sweet potato, banana, barley, wheat, rice, oat, sago, tapioca and sorghum. The starch utilized in the present invention is preferably derived from corn.

Starch phosphates are generally in the form of a white powder, whose elemental particle size ranges from 3 to 100 microns. The starches used in the composition of the invention may be chemically modified via one or more of the following reactions: pregelatinization, oxidation, crosslinking, esterification, heat treatments. In particular, the crosslinking reaction is preferred. This reaction is performed by crosslinking with functional agents capable of reacting with hydroxyl groups present in the starch molecules,

More preferably, the starch phosphate is chosen from:

(*i*) monostarch phosphate of formula (I),

$$\mathbf{A_m}\text{-O-PO-(O}\mathbf{X})_2 \qquad \text{(I)}$$

(*ii*) distarch phosphate of formula (II),

$$\mathbf{A_m}\text{-O-PO-(O}\mathbf{X})\text{-O-}\mathbf{A_m} \qquad \text{(II)}$$

(*iii*) tristarch phosphate of formula (III),

$$\mathbf{A_m}\text{-O-PO-(O-}\mathbf{A_m})_2 \qquad \text{(III)}$$

or a mixture thereof, wherein $\mathbf{A_m}$ represents starch; and $\mathbf{X}$ represents alkali metals, alkaline-earth metals, ammonium salts, amine salts, or ammonium salts derived from basic amino acids, more preferably sodium, potassium, calcium, magnesium, salts of monoethanolamine, diethanolamine, triethanolamine, 3-amino-1 ,2-propanediol, or ammonium salts derived from lysine, arginine, sarcosine, ornithine or citrulline.

Preferably the starch phosphate is chosen from distarch phosphates of formula (II), more preferably chosen from hydroxypropyl cassava distarch phosphate, cassava distarch phosphate, acetyl cassava distarch phosphate, hydroxypropyl distarch phosphate, or a mixture thereof; even more preferably the modified starch is hydroxypropyl distarch phosphate.

Mentions may be made of the distarch phosphates, for example, the products sold under the references Prejel VA-70-T AGGL (gelatinized hydroxypropyl cassava distarch phosphate), Prejel TK1 (gelatinized cassava distarch phosphate) and Prejel 200 (gelatinized acetyl cassava distarch phosphate) by the company Avebe, or Structure Zea (gelatinized hydroxypropyl corn distarch phosphate) by the company Akzo Nobel.

Preferably, the starch phosphates are present in the composition of the present invention in an amount of at least 0.10 wt%, more preferably at least 0.30 wt%, more preferably at least 0.50 wt%, even more preferably at least 0.75 wt%, most preferably at least 1.00 wt% relative to the total weight of the composition. Preferably, said amount is at most 25 wt%, more preferably at most 15 wt%, even more preferably at most 10 wt%, even more preferably at most 7 wt%, most preferably at most 5 wt%. Preferably said amount ranges from 0.5 wt% to 10 wt%, preferably from 0.75 wt% to 7 wt%, and better still from 1 wt% to 5 wt%.

In a preferred embodiment, the starch phosphate is present in the inventive composition in an amount of at least 0.25 wt%, more preferably at least 0.50 wt%, most preferably at least 1.00 wt%, relative to the total amount of the composition. Preferably said amount is at most 10.00 wt%, more preferably at most 5.00 wt%, most preferably at most 3.00 wt%. Preferably, the starch phosphate is present in an amount of between 0.25 wt% and 10.00 wt%, more preferably between 0.50 wt% and 5.00 wt%, most preferably between 1.00 wt% and 3.00 wt%.

The composition of the invention comprises Xanthan Gum (XG) and Locust Bean Gum (LBG) in a weight ratio XG:LBG of from 20:80 to 80:20, more preferably in a ratio of from 30:70 to 70:30; even more preferably in a ratio of from 40:60 to 60:40; most preferably in a ratio of from 45:55 to 55:45.

Xanthan gum is a polysaccharide secreted by the bacterium *Xanthomonas campestris,* and is commonly used as a food thickening agent (in salad dressings, for example) and a stabilizer (in cosmetic products, for example, to prevent ingredients from separating). It is composed of pentasaccharide repeat units, comprising glucose, mannose, and glucuronic acid in a molar ratio of typically 2:2:1. Xanthan gum may be produced by the fermentation of glucose, sucrose, or lactose. After a fermentation period, the polysaccharide is typically precipitated from a growth medium, e.g. with isopropyl alcohol, dried, and ground into a fine powder. Later, may be added to a liquid medium to form the gum.

The xanthan gum is preferably present in the inventive composition in an amount of at least 0.10 wt%, more preferably at least 0.15 wt%, most preferably at least 0.20 wt%, relative to the total amount of the composition. Preferably said amount is at most 0.60 wt%, more preferably at most 0.55 wt%, most preferably at most 0.50 wt%. Preferably, the xanthan gum is present in an amount of between 0.10 wt% and 0.60 wt%, more preferably between 0.15 wt% and 0.55 wt%, most preferably between 0.20 wt% and 0.5 wt%.

Locust bean gum (LBG, also known as carob gum, carob bean gum, or carobin) is typically used in the food industry as a thickening agent and a gelling agent. Locust bean gum is a galactomannan vegetable gum extracted from the seeds of the carob tree, mostly found in the Mediterranean region. The long pods that grow on the tree are used to make this gum. A typical process to extract LBG includes: kibbling the pods to separate the seed from the pulp; removing the skins of the seeds, e.g. by an acid treatment; splitting and gently milling the de-skinned seeds, which causes the brittle germ to break up while not affecting the more robust endosperm; separating the brittle germ from the endosperm, e.g. by sieving; and milling the separated endosperm, e.g. by a roller operation, to produce the final locust bean gum powder.

The LBG is preferably present in the inventive composition in an amount of at least 0.05 wt%, more preferably at least 0.10 wt%, most preferably at least 0.15 wt%, relative to the total amount of the composition. Preferably said amount is at most 0.60 wt%, more preferably at most 0.50 wt%, most preferably at most 0.40 wt%. Preferably, the LBG is present in an amount of between 0.05 wt% and 0.60 wt%, more preferably between 0.10 wt% and 0.50 wt%, most preferably between 0.15 wt% and 0.40 wt%.

Preferably, the XG and the LBG are present in the inventive composition in a total amount (i.e. the sum of the individual amounts) of at least 0.20 wt%, more preferably at least 0.25 wt%, most preferably at least 0.30 wt%, relative to the total amount of the composition. Preferably said total amount is at most 0.60 wt%, more preferably at most 0.55 wt%, most preferably at most 0.50 wt%. Preferably, said total amount is between 0.20 wt% and 0.60 wt%, more preferably between 0.25 wt% and 0.55 wt%, most preferably between 0.30 wt% and 0.50 wt%.

Preferably, the total amount of XG and LBG is present in the inventive composition in at least 10 wt% relative to the amount of starch phosphate (SP), more preferably at least 15 wt%, most preferably at least 20 wt%. Preferably, said total amount of XG and LBG is present in at most 40 wt% relative to the amount of starch phosphate, more preferably at most 30 wt%, most preferably at most 25 wt%. Preferably, said total amount of XG and LBG is between 5 wt% and 50 wt%, more preferably between 10 wt% and 40 wt%, more preferably between 15 wt% and 30 wt%, most preferably between 20 wt% and 25 wt% relative to the amount of the starch phosphate..

The composition of the invention comprises also at least one surfactant. The surfactants may be chosen from anionic surfactants, cationic surfactants, amphoteric or zwitterionic surfactants, nonionic surfactants, or a mixture thereof. Preferably the surfactants are chosen from anionic surfactants, nonionic surfactants, or a mixture thereof. More preferably the surfactants are chosen from anionic surfactants and nonionic surfactants having an HLB value of greater than or equal to 8, according to Griffin's method, examples of such nonionic surfactants being esters of polyols and of fatty acids possessing a saturated or unsaturated chain comprising, for example, from 8 to 24 carbon atoms and better still from 12 to 22 carbon atoms, and their oxyalkylenated derivatives, or a mixture thereof. The HLB value of the nonionic surfactant according to GRIFFIN is defined in J. Ploughshare. Cosm. Chem. 1954 (volume 5), pages 249-256.

Preferably, the nonionic surfactant has a HLB value between 8 and 20 according to Griffin's 20 method, at the temperature of 25 °C, more preferably between 10 and 15 according to Griffin's method, at the temperature of 25 °C.

Suitable anionic surfactants of the present invention may be chosen from alkyl sulphates, alkyl ether sulphates and their salts, alkyl sulphoacetates, alkyl sulphosuccinates, polypeptides which are obtained, for example, by condensation of a fatty chain with cereal amino acids and in particular wheat and oat amino acids, amino acid derivatives, sulphonates, for example a-olefin sulphonates, isethionates, in particular acyl isethionates, phosphoric esters and salts thereof, or a mixture thereof.

Preferably, the surfactant used in the inventive composition is a non-ionic surfactant, which is an ester of polyols and of fatty acids possessing a saturated or unsaturated chain comprising, for example, from 8 to 24 carbon atoms and better still from 12 to 22 carbon atoms, and their oxyalkylenated derivatives. Preferably, the nonionic surfactant is chosen from esters of glycerol and of a $C_8$-$C_{24}$ fatty acid, and their oxyalkylenated derivatives; esters of polyethylene glycol and of a $C_8$-$C_{24}$ fatty acid, and their oxyalkylenated derivatives; esters of sorbitol and of a $C_8$-$C_{24}$ fatty acid, and their oxyalkylenated derivatives; ethers of fatty alcohols; ethers of a sugar and of $C_8$-$C_{24}$ fatty alcohols, and their mixtures. Mention may in particular be made, as ester of glycerol and of a $C_8$-$C_{24}$ fatty acid, of glyceryl stearate (glyceryl mono-, di- and/or tristearate) (CTFA name: glyceryl stearate) or glyceryl ricinoleate, and their mixtures, for example the product sold under the trade name Cerasynt SD by ISP. Preferably the nonionic surfactant is glyceryl stearate. Polyethylene glycol esters of fatty acids that may especially be mentioned include polyethylene glycol stearate (polyethylene glycol monostearate, distearate and/or tristearate) and more especially polyethylene glycol 50 OE monostearate (CTFA name: PEG-50 stearate) and polyethylene glycol 100 OE monostearate (CTFA name: PEG-100 stearate), and mixtures thereof. Mixtures of these surfactants may also be used, for instance the product containing glyceryl stearate and PEG-100 stearate, sold under the name Arlacel 165 by the company Uniqema, and the product containing glyceryl stearate (glyceryl monodistearate) and potassium stearate, sold under the name Tegin by the company 20 Goldschmidt (CTFA name: glyceryl stearate SE). Examples of fatty alcohol ethers that may be mentioned include polyethylene glycol ethers of fatty alcohols containing from 8 to 30 carbon atoms and especially from 10 to 22 carbon atoms, such as polyethylene glycol ethers of cetyl alcohol, of stearyl alcohol or of cetearyl alcohol (mixture of cetyl alcohol and stearyl alcohol). Examples that may be mentioned include ethers comprising from 1 to 200 and preferably from 2 to 100 oxyethylene groups, such as those of CTFA name Ceteareth-20 and Ceteareth-30, and mixtures thereof. By way of example of sugar mono- or polyalkyl esters or ethers, mention may be made of the methylglucose isostearate sold under the name Isolan-IS by the company Degussa Goldschmidt, or the sucrose distearate sold under the name Crodesta F50 by the company Croda, and the sucrose stearate sold under the name Ryoto sugar ester S 1570 by the company Mitsubishi Kagaku Foods.

Preferably, the anionic surfactant is chosen from phosphoric esters and salts, in particular phosphoric esters and salts of $C_2$-$C_{30}$ linear or branched, saturated or unsaturated alcohols, more preferably phosphoric esters and salts of $C_4$-$C_{24}$, even more preferably $C_6$-$C_{18}$, for example $C_{16}$ or $C_{18}$ linear or branched, saturated or unsaturated alcohols, Mentions may be made of the preferred anionic surfactant, such as DEA oleth-10 phosphate (Crodafos N 10N from the company Croda) or potassium cetyl phosphate (Amphisol® K from DSM Nutritional Products), more preferably the anionic surfactant is potassium cetyl phosphate.

More preferably, the anionic surfactant is chosen from made of lipoamino acids and salts thereof, such as monosodium and disodium acylglutamates, for instance the monosodium stearoyl glutamate sold under the name Amisoft HS-11P and the disodium stearoyl glutamate sold under the name Amisoft HS-21P by the company Ajinomoto.

Preferably, the inventive composition comprises a surfactant that has foaming properties, i.e. it forms a foam when a gas is introduced into aqueous medium containing thereof. More preferably, said surfactant having foaming capabilities (hereinafter "foaming surfactant") is a anionic surfactant: salt of fatty acid and monovalent cation such as sodium stearate; alkyl esters or ethers of sulfuric, sulfonic acid or sulfosuccinic acids, such as sodium lauryl sulfate or sodium laureth sulfate, sodium c14-16 olefin sulfonate, ammonium lauryl sulfosuccinate; amino acids derivatives such as sodium lauryl sarcosinate, Sodium Methyl Cocoyl Taurate, or monosodium stearoyl glutamate.

In a preferred embodiment, the inventive composition comprises a surfactant which is a blend of an ester of glycerol and of a $C_8$-$C_{24}$ fatty acid (e.g. glyceryl stearate) and a lipoamino acid or salt of a sugar mono- or polyalkyl ester or ether (e.g. monosodium stearoyl glutamate).

The surfactant is preferably present in the inventive composition in an amount of at least 0.1 wt%, more preferably at least 1.0 wt%, most preferably at least 2.0 wt%, relative to the total amount of the composition. Preferably said amount is at most 10.0 wt%, more preferably at most 7.0 wt%, most preferably at most 5.0 wt%. Preferably, the surfactant is present in an amount of between 0.1 wt% and 10.0 wt%, more preferably between 1.0 wt% and 7.0 wt%, most preferably between 2.0 wt% and 5.0 wt%.

When a foaming surfactant is used, preferably the surfactant is present in an amount of at least 0.1 wt%, more preferably at least 0.3 wt%, most preferably at least 0.5 wt%, relative to the total amount of the composition. Preferably said amount is at most 3 wt%, more preferably at most 2 wt%, most preferably at most 1.5 wt%. Preferably, the foaming surfactant is present in an amount of between 0.1 wt% and 3 wt%, more preferably between 0.3 wt% and 2 wt%, most preferably between 0.5 wt% and 1.5 wt%.

The inventive compositions may further contain one or more of adjuvants that are common in the cosmetic and dermatological fields: hydrophilic or lipophilic gelling agents and/or thickeners; moisturizers; emollients; hydrophilic or lipophilic active agents; free-radical scavengers; sequestrants; antioxidants; preserving agents; basifying or acidifying agents; fragrances; film-forming agents; fillers; and mixtures thereof. The amounts of these various adjuvants are those conventionally used in the fields under consideration. In particular, the amounts of active agents vary according to the desired objective and are those conventionally used in the fields under consideration, and for example from 0.1% to 20%, and preferably from 0.5% to 10% by weight of the total weight of the composition.

The invention further relates to a process for manufacturing the inventive composition, comprising the steps of:

a. Providing an aqueous phase comprising the at least one starch phosphate, the surfactant, the Xantan Gum (XG) and the Locust Bean Gum (LBG) wherein the ratio XG:LBG is from 20:80 to 80:20;
b. Providing a fatty phase;
c. Foaming the aqueous phase and subsequently adding and the fatty phase to the foamed aqueous phase to obtain a foamed composition;
d. Cooling the foamed composition with a cooling rate of at least 1.0 °C/minute.

In a preferred embodiment, the invention relates to a process for manufacturing the inventive composition, comprising the steps of:

a. Providing an aqueous phase comprising the at least one starch phosphate, the surfactant, the Xantan Gum (XG) and the Locust Bean Gum (LBG) wherein the ratio XG:LBG is from 20:80 to 80:20; wherein said aqueous phase has a temperature of at least 70°C, more preferably at least 80°C;
b. Providing a fatty phase having a temperature of at least 70°C, more preferably at least 80°C;
c. Foaming the aqueous phase and subsequently adding and the fatty phase to the foamed aqueous phase to obtain a foamed composition;
d. Cooling the foamed composition to a first temperature $T_1$ with a cooling rate of at least 1.0 °C/minute and subsequently cooling the foam to a second temperature $T_2$ with a cooling rate of less than 1 °C/minute, wherein $T_1 > T_2$.

Preferably, at step a. the starch phosphate, XG and LBG are dispersed under stirring in an aqueous medium to form the aqueous phase. The aqueous medium may be water or any medium containing water, preferably in an amount of at least 50 wt% relative to the total weight of said medium. The surfactant is preferably added after dispersing the above three components in the aqueous medium; although it may also be added before.

The aqueous phase may be foamed by incorporating a gas therein, the gas being any suitable gas e.g. air, $CO_2$, etc. The gas may be incorporated by any suitable manner including, but not limited to, simple mixing in contact with an atmosphere containing the gas or even ambient conditions, aeration, homogenization or other methods of injecting a gas into or through the aqueous phase and by use of gas expansion devices.

In one embodiment, the aqueous phase is foamed by mechanically incorporating the gas therein by stirring said aqueous phase with a whipping machine, e.g. as commonly found in any store selling home appliances. The stirring is preferably carried out at between 800 and 1700 rpm, more preferably between 900 and 1600 rpm, most preferably between 1000 and 1500 rpm. A suitable stirring time is at least 3.0 seconds per gram of aqueous phase (sec/g), more preferably at least 4 sec/g, most preferably at least 5 sec/g. The inventors observed that the stirring introduces the correct amount of gas and ensures that optimally distributed air pockets having optimum sizes are obtained.

In another embodiment of the method of the invention (hereinafter the "inventive method"), the introduction of the gas, preferably air, into the first mixture is carried out in an expansion device comprising a mixing head having a rotor and a stator, such as, for example, the "Minimondo-type Mondomixer" commercially available from Mondomix. The mixture is transported via a pump into the expansion head, where the mixture and the gas are simultaneously injected and homogeneously mixed by virtue of the cutting action of the lugs of the rotor and stator of the device, which provide even distribution of the gas in the mixture. The speed of the rotor of the device, the temperature of the vessel, gas pressure and flow rate of the gas are appropriately regulated. The pressure of the mixing head is regulated by a pressure regulator. The flow rate of the low density cosmetic formulation at the outlet of the device depends on the rate of the pump at the vessel outlet.

Preferably, the fatty phase is added to the foamed aqueous phase under agitation, more preferably under stirring with between 200 and 1000 rpm, more preferably between 300 and 900 rpm, most preferably between 400 and 800 rpm. Preferably a whipping machine is used to provide the stirring. Preferably, the fatty phase in added at a rate of between 0.5 and 10.0 grams/minute (g/min), more preferably between 1.0 and 5.0 g/min, most preferably between 1.5 and 3.0 g/min. The inventors observed that said agitation and rate of fatty phase addition to the aqueous phase ensure that the oil phase is optimally distributed within the aqueous phase with oil droplets having an optimum size.

Preferably, the foamed composition is mixed under stirring with between 200 and 1000 rpm, more preferably between 300 and 900 rpm, most preferably between 400 and 800 rpm. Preferably, the mixing time is at least 1.0 seconds per gram of the foamed composition, more preferably at least 2.0 sec/g, most preferably at least 3.0 sec/g. Said mixing time is preferably at most 20.0 seconds per gram of the foamed composition, more preferably at most 10.0 sec/g, most preferably at most 5.0 sec/g.

The foamed composition is cooled to a temperature $T_1$ with a cooling rate of at least 1.0 °C/minute, more preferably at least 1.5 °C/minute, most preferably at least 1.8 °C/minute. Preferably the temperature $T_1$ is at least 40°C, more preferably at least 45°C.

After the first cooling, the foamed composition is cooled to a second temperature $T_2$ with a second cooling rate of less than 1 °C/minute, more preferably at most 0.8 °C/minute, most preferably at most 0.5 °C/minute.

The obtained foam, had optimum stability, being light and providing a fresh and unique sensation when applied to e.g. the skin. The stability of the foam is defined by its ability to retain its structure (number of air pockets, size of the air pockets) over time. Foams are generally thermodynamically unstable their stability depending greatly on that of the walls which separate the bubbles, i.e. the interfaces. The present inventors observed that the foams of the present invention have optimum characteristics which in turn confer stability thereof. The air pockets in the inventive composition can generally have a size ranging from about 20 $\mu$m to about 6000 $\mu$m, preferably from about 100 $\mu$m to about 3000 $\mu$m. In general, it is advantageous from an aesthetic perspective that at least a portion of the bubbles are visible without magnification.

The invention further relates to methods of treating hair, nails and/or skin with the inventive composition. Such methods generally include providing a hair or skin substrate to be treated, preferably skin, and contacting the substrate with the inventive composition. Methods in accordance with this aspect of the invention can include application of the inventive composition to the substrate with any of direct application to the dry substrate, wetting, lathering, rinsing, allowing to stand followed by rinsing, blow-drying, dyeing, lightening, highlighting, perming, relaxing, making up skin, nails, lips and/or hair, moisturizing and massaging. Any of these additional steps or combinations thereof can be carried out prior to, simultaneously with or subsequent to the application of the inventive composition.

The inventive composition can be used for a non-therapeutic process, such as a cosmetic process or method, for making up/caring for the keratin materials, such as the skin, in particular the face and the hands, by being applied to the skin, especially the face and the hands. The present invention also relates to a use of the inventive composition as it is or in cosmetic product for making up/caring for/cleansing/make up removing products for the skin, especially for the face and the hands.

The examples that follow are aimed at illustrating the compositions and processes according to this invention, but are not in any way a limitation of the scope of the invention.

METHODS OF MEASUREMENT

- **Density:** the density of a foam is the ratio of the weight of a given volume of the foam to the weight of the same volume of water under defined temperature conditions, which for the present invention was set at 20°C. The density may be measured by using always the same container. Knowing the density of demineralized water, and the weight of it, the exact volume of the container can be calculated as follows:

  - the container is weighed before and after filling with demineralized water. Volume of the container is calculated using formula:

$$volume\ container(T\ °C) = \frac{mass\ water}{density\ water}$$

  - the density of water was considered as being 1 g/cm$^3$, thus
    *volume container*(*T* °C) = *mass water* (*in grams*) which leads to a density of the foam of:

$$density\ foam(T\ °C) = \frac{mass\ foam}{mass\ water}$$

  in the given container. The mass is expressed in grams and the volume in cubic centimeters.

EXAMPLE 1 - Method of producing a foamed composition

Glycerin and Geogard 221 were added under stirring (500 rpm, 5 minutes) in demineralized water. Subsequently, the stabilizers were dispersed under stirring (2000 rpm, 10 minutes) in the aqueous phased until homogeneous. The surfactant was subsequently added under stirring (250 rpm, 10 minutes).

The water phase was heated to 80°C while mildly stirring (250 rpm, 30 minutes) after which the heated water phase was foamed with a cooking mixer operating at 1200rpm for 45 seconds. Subsequently the fatty phase heated up to 80°C was added while stirring with 1200 rpm over a period of time of 45 seconds. The stirring was continued at 600 rpm for 80 seconds.

Immediately after mixing, the foamed composition was placed in a freezer set at minus 18°C for 5 minutes to cool said composition to 46°C, after which said composition was placed in a refrigerator set at 4°C for 20 minutes to reach a temperature of 36°C. The composition was subsequently packed in appropriated jars.

The ingredients reported in Table 1 were used. The density of the obtained foam was 0.64 g/cm$^3$.

The foam had an appealing appearance resembling whipped cream having a high degree of whiteness, which is typically very well received by consumers. Air bubbles were well entrapped giving a highly satisfying sensory effect. A "cracking" noise could be heard when the bursting of the air pockets happened during application of the foam on the skin. In addition, spreadability of the foam was highly satisfying, while texture presented a creamy and cocooning effect.

The composition was analyzed in terms of stability. Organoleptic properties were monitored for various periods of time ranging from one day (control check point (CCP): D+1), to more than one week (CCP: W+1), e.g. two weeks (CCP: W+2), one month (CCP: 1M), two months (CCP: 2M), three months (CCP: 3M), four months (CCP: 4M) and five months (CCP: 5M) after manufacture, in different conditions: room temperature stored in the absence of light, room temperature stored in the presence of sunlight, 4°C in the absence of light, 50°C in the absence of light.

At all CCPs the foamed compositions according to the invention showed satisfying properties in terms of appearance and volume of the foam until a certain time. The sample stored at 50°C showed a satisfying behavior up to 1M: volume of the foam remained at the same height, due to the fact that air bubbles were well entrapped.

The samples stored at room temperature in presence of sunlight, room temperature in the absence of light, and at 4°C showed a satisfying behavior up to 5M: volume of the foam remained at the same height, due to the fact that air bubbles were well entrapped, and sensory remained satisfying. A "cracking" noise could be heard when the bursting of the air pockets happened. In addition, spreadability of the foam was satisfying, while texture presented a creamy and cocooning effect.

COMPARATIVE EXPERIMENT 1

Example 2 was repeated with the difference that instead of the (corn) starch phosphate a native tapioca starch was used. The ingredients are mentioned in Table 1.

Sensory of the trial was rather acceptable but the stability at 50 °C was not good with loss of volume at 50°C.

COMPARATIVE EXPERIMENT 2

Example 1 was repeated with the difference that no starch was used and the level of use of the LBG and XG was increased. The ingredients are mentioned in Table 1.

Sensory of the trial was not pleasant with the samples having a jelly effect and lacking spreadability. Stability at 50°C was less good than the sample of the Example 1 but still acceptable.

COMPARATIVE EXPERIMENT 3

Example 2 was repeated with the difference that instead of the Xanthan and LBG a iota carrageenan was used. The ingredients are mentioned in Table 1.

Here, sensory of the trial was acceptable. Air bubbles were well entrapped right after manufacture. The sample however lacked stability at 50°C where the air bubbles were almost immediately lost.

Table 1

| Phase | Ingredients | | Ex.1 % | Ex.2 % | C.Ex.1 % | C.Ex.2 % | C.Ex.3 % |
|---|---|---|---|---|---|---|---|
| | Brand name | INCI name | | | | | |
| Aqueous phase | Demi water | Water | 66.8 | 67 | 67 | 68.1 | 67 |
| | Refined Glycerin | Glycerin | 7 | 7 | 7 | 7 | 7 |
| | Geogard 221 | Benzyl alcohol, dehydroacetic | 0.5 | 0.5 | 0.5 | 0.5 | 0.5 |
| | | acid and water | | | | | |
| | Satiaxane CX 930 | Xanthan gum | 0.22 | 0.11 | 0.11 | 0.33 | |
| | Viscogum BRC 13/252 | Locust Bean Gum | 0.18 | 0.09 | 0.09 | 0.27 | |
| | C*HiForm A12747 | HP Starch phosphate (maize) | 1.5 | 2 | | | 2 |
| | Satiagel VPC 508 | Carrageenan | | | | | 0.2 |
| | C* CreamGel 70001 | Tapioca starch | | | 2 | | |
| | Amisoft HS11P | Sodium stearoyl glutamate | 0.8 | 0.8 | 0.8 | 0.8 | 0.8 |
| Fatty phase | Sugin | Glyceryl stearate | 2.5 | 2.5 | 2.5 | 2.5 | 2.5 |
| | Miraceti | Cetyl esters | 2.5 | 2.5 | 2.5 | 2.5 | 2.5 |
| | Orbignya Cetyla | Cetyl babassuate | 5 | 5 | 5 | 5 | 5 |
| | Monaco USP Cocoa Butter | Cocoa butter | 2.5 | 2.5 | 2.5 | 2.5 | 2.5 |
| | Sweet almond oil | Sweet almond oil | 2.5 | 2.5 | 2.5 | 2.5 | 2.5 |
| | Tegasoft CT | Caprilic capric triglycerides | 7.5 | 7.5 | 7.5 | 7.5 | 7.5 |
| | D alpha tocopherol alcohol | tocopherol | 0.5 | 0.5 | 0.5 | 0.5 | 0.5 |

**Claims**

1. An oil-in-water composition comprising a fatty phase dispersed in an aqueous phase, said composition being in the form of a foam and having a density of between 0.3 to 0.9, the composition comprising:

    a. At least one starch phosphate;
    b. Xanthan Gum (XG) and Locust Bean Gum (LBG) in a ratio XG:LBG from 20:80 to 80:20; and
    c. At least one surfactant.

2. The composition of claim 1, wherein the ratio XG:LBG is from 30:70 to 70:30, more preferably from 40:60 to 60:40.

3. The composition of any one of the preceding claims, wherein the density of the composition is from 0.4 to 0.8, more preferably from 0.5 to 0.7.

4. The composition of any one of the preceding clams, wherein the fatty phase is present in an amount ranging from 1 wt% to 90 wt%, preferably from 5 wt% to 70 wt%, more preferably from 10 wt% to 30 wt%, most preferably from 15 wt% to 25 wt%, relative to the total weight of the composition.

5. The composition of any one of the preceding claims, wherein the aqueous phase is present in an amount ranging from 10 wt% to 99 wt%, preferably from 20 wt% to 90 wt%, more preferably from 30 wt% to 85 wt%, most preferably from 50 wt% to 80 wt%, relative to the total weight of the composition.

6. The composition of any one of the preceding claims, wherein the modified starch is chosen from monostarch phosphate of formula (I),

$$\mathbf{A_m}\text{-O-PO-(O}\mathbf{X})_2 \qquad (I)$$

distarch phosphate of formula (II),

$$\mathbf{A_m}\text{-O-PO-(O}\mathbf{X})\text{-O-}\mathbf{A_m} \qquad (II)$$

tristarch phosphate of formula (III),

$$\mathbf{A_m}\text{-O-PO-(O-}\mathbf{A_m})_2 \qquad (III)$$

or a mixture thereof,
wherein

$\mathbf{A_m}$ represents starch;
$\mathbf{X}$ represents alkali metals, alkaline-earth metals, ammonium salts, amine salts, or ammonium salts derived from basic amino acids, more preferably sodium, potassium, calcium, magnesium, salts of monoethanolamine, diethanolamine, triethanolamine, 3-amino-1,2-propanediol, or ammonium salts derived from lysine, arginine, sarcosine, ornithine or citrulline;
preferably the starch phosphate is chosen from distarch phosphates of formula (II), more preferably chosen from hydroxypropyl cassava distarch phosphate, cassava distarch phosphate, acetyl cassava distarch phosphate, hydroxypropyl distarch phosphate, or a mixture thereof; even more preferably the modified starch is hydroxypropyl distarch phosphate.

7. The composition of any one of the preceding claims, wherein the starch phosphate is present in an amount ranging from 0.5 wt% to 10 wt%, preferably from 1 wt% to 7 wt%, and better still from 2 wt% to 5 wt%, relative to the total weight of the composition.

8. The composition of any one of the preceding claims, wherein the Xanthan Gum is present in an amount ranging from 0.1 wt% to 0.6 wt%, preferably from 0.15 wt% to 0.55 wt%, more preferably from 0.2 wt% to 0.5 wt%, relative to the total weight of the composition.

9. The composition of any one of the preceding claims, wherein the Locust Bean Gum is present in an amount ranging from 0.05 wt% to 0.6 wt%, preferably from 0.1 wt% to 0.5 wt%, more preferably from 0.15 wt% to 0.4 wt%, relative to the total weight of the composition.

10. The composition of any one of the preceding claims, wherein the surfactant is a non-ionic surfactant having a HLB value between 8 and 20 according to Griffin's 20 method, at the temperature of 25 °C, more preferably between 10 and 15 according to Griffin's method, at the temperature of 25 °C.

11. The composition of any one of the preceding claims, wherein the surfactant comprises a blend of an ester of glycerol and of a $C_8$-$C_{24}$ fatty acid (e.g. glyceryl stearate) and a lipoamino acid or salt of a sugar mono- or polyalkyl ester or ether (e.g. monosodium stearoyl glutamate).

12. The composition of any one of the preceding claims, wherein the surfactant that has foaming properties.

13. The composition of any one of the preceding claims, wherein the surfactant is present in an amount ranging from 0.05 wt% to 10 wt%, preferably from 0.1 wt% to 5 wt%, relative to the total weight of the composition.

14. A process for manufacturing the composition of any one of the preceding claims, comprising the steps of:

a. Providing an aqueous phase comprising the at least one starch phosphate, the surfactant, the Xantan Gum (XG) and the Locust Bean Gum (LBG) wherein the ratio XG:LBG is from 20:80 to 80:20;

b. Providing a fatty phase;

c. Foaming the aqueous phase and subsequently adding and the fatty phase to the foamed aqueous phase to obtain a foamed composition;

d. Cooling the foamed composition with a cooling rate of at least 1.0 °C/minute.

**Patentansprüche**

1. Öl-in-Wasser-Zusammensetzung, die eine in einer wässrigen Phase dispergierte Fettphase umfasst, wobei die Zusammensetzung in Form eines Schaums ist und eine Dichte zwischen 0,3 und 0,9 aufweist, wobei die Zusammensetzung umfasst:

a. zumindest ein Stärkephosphat;

b. Xanthangummi (XG) und Johannisbrotkernmehl (LBG) in einem Verhältnis XG:LBG von 20:80 bis 80:20; und

c. zumindest ein Tensid.

2. Zusammensetzung nach Anspruch 1, wobei das Verhältnis XG:LBG von 30:70 bis 70:30, bevorzugter von 40:60 bis 60:40 beträgt.

3. Zusammensetzung nach einem der vorstehenden Ansprüche, wobei die Dichte der Zusammensetzung von 0,4 bis 0,8, bevorzugter von 0,5 bis 0,7 beträgt.

4. Zusammensetzung nach einem der vorstehenden Ansprüche, wobei die Fettphase in einer Menge im Bereich von 1 Gew.-% bis 90 Gew.-%, vorzugsweise von 5 Gew.-% bis 70 Gew.-%, bevorzugter von 10 Gew.-% bis 30 Gew.-%, besonders bevorzugt von 15 Gew.-% bis 25 Gew.-%, bezogen auf das Gesamtgewicht der Zusammensetzung vorhanden ist.

5. Zusammensetzung nach einem der vorstehenden Ansprüche, wobei die wässrige Phase in einer Menge im Bereich von 10 Gew.-% bis 99 Gew.-%, vorzugsweise von 20 Gew.-% bis 90 Gew.-%, bevorzugter von 30 Gew.-% bis 85 Gew.-%, besonders bevorzugt von 50 Gew.-% bis 80 Gew.-%, bezogen auf das Gesamtgewicht der Zusammensetzung vorhanden ist.

6. Zusammensetzung nach einem der vorstehenden Ansprüche, wobei die modifizierte Stärke ausgewählt ist aus Monostärkephosphat der Formel (I),

$$A_m\text{-O-PO-(O}X)_2 \qquad \text{(I)}$$

Distärkephosphat der Formel (II),

$$A_m\text{-O-PO-(O}X)\text{-O-}A_m, \qquad \text{(II)}$$

Tristärkephosphat der Formel (III),

$$A_m\text{-O-PO-(O-}A_m)_2 \qquad \text{(III)}$$

oder ein Gemisch davon,
wobei

$A_m$ Stärke repräsentiert;
$X$ Alkalimetalle, Erdalkalimetalle, Ammoniumsalze, Aminsalze oder Ammoniumsalze abgeleitet von basischen Aminosäuren, bevorzugter Natrium, Kalium, Calcium, Magnesium, Salze von Monoethanolamin, Diethanolamin, Triethanolamin, 3-Amino-1,2-Propanediol oder Ammoniumsalze abgeleitet von Lysin, Arginin, Sarkosin, Ornithin oder Citrullin repräsentiert;
vorzugsweise ist das Stärkephosphat ausgewählt aus Distärkephosphaten der Formel (II), bevorzugter ausgewählt aus Hydroxypropylcassavadistärkephosphat, Cassavadistärkephosphat, Acetylcassavadistärkephosphat, Hydroxypropyldistärkephosphat oder einem Gemisch davon; noch bevorzugter ist die modifizierte Stärke Hydroxypropyldistärkephosphat.

**7.** Zusammensetzung nach einem der vorstehenden Ansprüche, wobei das Stärkephosphat in einer Menge im Bereich von 0,5 Gew.-% bis 10 Gew.-%, vorzugsweise von 1 Gew.-% bis 7 Gew.-% und noch besser von 2 Gew.-% bis 5 Gew.-%, bezogen auf das Gesamtgewicht der Zusammensetzung vorhanden ist.

**8.** Zusammensetzung nach einem der vorstehenden Ansprüche, wobei der Xanthangummi in einer Menge im Bereich von 0,1 Gew.-% bis 0,6 Gew.-%, vorzugsweise von 0,15 Gew.-% bis 0,55 Gew.-%, bevorzugter von 0,2 Gew.-% bis 0,5 Gew.-%, bezogen auf das Gesamtgewicht der Zusammensetzung vorhanden ist.

**9.** Zusammensetzung nach einem der vorstehenden Ansprüche, wobei das Johannisbrotkernmehl in einer Menge im Bereich von 0,05 Gew.-% bis 0,6 Gew.-%, vorzugsweise von 0,1 Gew.-% bis 0,5 Gew.-%, bevorzugter von 0,15 Gew.-% bis 0,4 Gew.-%, bezogen auf das Gesamtgewicht der Zusammensetzung vorhanden ist.

**10.** Zusammensetzung nach einem der vorstehenden Ansprüche, wobei das Tensid ein nichtionisches Tensid ist, das einen HLB-Wert zwischen 8 und 20 nach dem Griffin's 20 Verfahren aufweist, bei der Temperatur von 25 °C, bevorzugter zwischen 10 und 15 nach dem Griffin's Verfahren, bei der Temperatur von 25 °C.

**11.** Zusammensetzung nach einem der vorstehenden Ansprüche, wobei das Tensid eine Mischung aus einem Ester von Glyzerin und von einer $C_8$-$C_{24}$-Fettsäure (z.B. Glycerylstearat) und einer Lipoaminosäure oder Salz eines Zuckermono- oder polyalkylesters oder-ethers (z.B. Mononatriumstearoylglutamat) umfasst.

**12.** Zusammensetzung nach einem der vorstehenden Ansprüche, wobei das Tensid, das schäumende Eigenschaften aufweist.

**13.** Zusammensetzung nach einem der vorstehenden Ansprüche, wobei das Tensid in einer Menge im Bereich von 0,05 Gew.-% bis 10 Gew.-%, vorzugsweise von 0,1 Gew.-% bis 5 Gew.-%, bezogen auf das Gesamtgewicht der Zusammensetzung vorhanden ist.

**14.** Verfahren zur Herstellung der Zusammensetzung nach einem der vorstehenden Ansprüche, umfassend die Schritte von:

a. Bereitstellen einer wässrigen Phase, die zumindest ein Stärkephosphat, das Tensid, den Xanthangummi (XG) und das Johannisbrotkernmehl (LBG) umfasst, wobei das Verhältnis XG:LBG von 20:80 bis 80:20 ist;
b. Bereitstellen einer Fettphase;
c. Schäumen der wässrigen Phase und anschließend Hinzufügen und der Fettphase zu der geschäumten wässrigen Phase, um eine geschäumte Zusammensetzung zu erhalten;
d. Kühlen der geschäumten Zusammensetzung mit einer Kühlrate von zumindest 1,0 °C/Minute.

**Revendications**

**1.** Composition huile-dans-eau comprenant une phase grasse dispersée dans une phase aqueuse, ladite composition étant sous la forme d'une mousse et ayant une densité entre 0,3 à 0,9, la composition comprenant :

a. Au moins un phosphate d'amidon ;
b. De la Gomme de Xanthane (XG) et de la Gomme de Caroube (LBG) dans un rapport XG:LBG de 20:80 à 80:20 ; et
c. Au moins un tensioactif.

**2.** Composition de la revendication 1, dans laquelle le rapport XG:LBG est de 30:70 à 70:30, plus préférentiellement de 40:60 à 60:40.

**3.** Composition de l'une quelconque des revendications précédentes, dans laquelle la densité de la composition est de 0,4 à 0,8, plus préférentiellement de 0,5 à 0,7.

**4.** Composition de l'une quelconque des revendications précédentes, dans laquelle la phase grasse est présente en une quantité allant de 1 % en poids à 90 % en poids, de préférence de 5 % en poids à 70 % en poids, plus préférentiellement de 10 % en poids à 30 % en poids, le plus préférentiellement de 15 % en poids à 25 % en poids, par rapport au poids total de la composition.

**5.** Composition de l'une quelconque des revendications précédentes, dans laquelle la phase aqueuse est présente en une quantité allant de 10 % en poids à 99 % en poids, de préférence de 20 % en poids à 90 % en poids, plus préférentiellement de 30 % en poids à 85 % en poids, le plus préférentiellement de 50 % en poids à 80 % en poids, par rapport au poids total de la composition.

**6.** Composition selon l'une quelconque des revendications précédentes, dans laquelle l'amidon modifié est choisi parmi le phosphate de mono-amidon de formule (I),

$$\mathbf{A}_m\text{-O-PO-(O}\mathbf{X})_2 \qquad (I)$$

phosphate de di-amidon de formule (II),

$$\mathbf{A}_m\text{-O-PO-(O}\mathbf{X})\text{-O-}\mathbf{A}_m \qquad (II)$$

phosphate de tri-amidon de formule (III),

$$\mathbf{A}_m\text{-O-PO-(O-}\mathbf{A}_m)_2 \qquad (III)$$

ou un mélange de ceux-ci,
dans laquelle

$\mathbf{A}_m$ représente l'amidon ;
$\mathbf{X}$ représente des métaux alcalins, des métaux alcalino-terreux, des sels d'ammonium, des sels d'amines ou des sels d'ammonium dérivés d'acides aminés basiques, plus préférentiellement de sodium, de potassium, de calcium, de magnésium, des sels de mono-éthanolamine, de di-éthanolamine, de tri-éthanolamine, de 3-amino-1,2-propanediol ou des sels d'ammonium dérivés de la lysine, de l'arginine, de la sarcosine, de l'ornithine ou de la citrulline ;
de préférence le phosphate d'amidon est choisi parmi les phosphates de di-amidon de formule (II), plus préférentiellement choisis parmi du phosphate de di-amidon de manioc hydroxypropylé, du phosphate de di-amidon de manioc, du phosphate de di-amidon de manioc acétylé, du phosphate de di-amidon hydroxypropylé, ou un mélange de ceux-ci ; encore plus préférentiellement l'amidon modifié est de phosphate de di-amidon hydroxypropylé.

**7.** Composition de l'une quelconque des revendications précédentes, dans laquelle le phosphate d'amidon est présent en une quantité allant de 0,5 % en poids à 10 % en poids, de préférence de 1 % en poids à 7 % en poids, et encore mieux de 2 % en poids à 5 % en poids, par rapport au poids total de la composition.

**8.** Composition de l'une quelconque des revendications précédentes, dans laquelle la Gomme Xanthane est présente en une quantité allant de 0,1 % en poids à 0,6 % en poids, de préférence de 0,15 % en poids à 0,55 % en poids, plus préférentiellement de 0,2 % en poids à 0,5 % en poids, par rapport au poids total de la composition.

**9.** Composition de l'une quelconque des revendications précédentes, dans laquelle la Gomme de Caroube est présente en une quantité allant de 0,05 % en poids à 0,6 % en poids, de préférence de 0,1 % en poids à 0,5 % en poids, plus préférentiellement de 0,15 % en poids à 0,4 % en poids, par rapport au poids total de la composition.

**10.** Composition de l'une quelconque des revendications précédentes, dans laquelle le tensioactif est un tensioactif non-ionique ayant une valeur de HLB comprise entre 8 et 20 selon la méthode 20 de Griffin, à la température de 25 °C, plus préférentiellement entre 10 et 15 selon la méthode de Griffin, à la température de 25 °C.

**11.** Composition de l'une quelconque des revendications précédentes, dans laquelle le tensioactif comprend un mélange d'un ester de glycérol et d'un acide gras en $C_8$ à $C_{24}$ (par exemple le stéarate de glycéryle) et un lipoaminoacide ou un sel d'un éther ou ester mono- ou polyalkylique de sucre (par exemple, le glutamate de stéaroyle monosodique).

**12.** Composition de l'une quelconque des revendications précédentes, dans laquelle le tensioactif qui a des propriétés moussantes.

**13.** Composition de l'une quelconque des revendications précédentes, dans laquelle le tensioactif est présent en une quantité allant de 0,05 % en poids à 10 % en poids, de préférence de 0,1 % en poids à 5 % en poids, par rapport au poids total de la composition.

14. Procédé de fabrication de la composition de l'une quelconque des revendications précédentes, comprenant les étapes de :

a. Fournir une phase aqueuse comprenant le au moins un phosphate d'amidon, le tensioactif, la Gomme de Xantane (XG) et la Gomme de Caroube (LBG), dans laquelle le rapport XG:LBG est de 20:80 à 80:20 ;
b. Fournir une phase grasse ;
c. Faire mousser la phase aqueuse et ensuite ajouter et la phase grasse à la phase aqueuse moussée pour obtenir une composition moussée ;
d. Refroidir la composition moussée avec une vitesse de refroidissement d'au moins 1,0 °C/minute.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013087926 A

- WO 2015096115 A

**Non-patent literature cited in the description**

- *J. Ploughshare. Cosm. Chem.,* 1954, vol. 5, 249-256